# EUROPEAN PATENT SPECIFICATION

(11) **EP 4 370 196 B1**
(45) Date of publication and mention of the grant of the patent: **19.03.2025**
(21) Application number: 22731821.9
(22) Date of filing: 01.06.2022
(51) Int. Cl.: A61N 1/36, A61N 1/04

(54) **DEVICE FOR CARRYING OUT DIATHERMIC AND MUSCLE STIMULATION TREATMENTS**
VORRICHTUNG ZUR DURCHFÜHRUNG DIATHERMISCHER UND MUSKELSTIMULATIONSBEHANDLUNGEN
DISPOSITIF POUR LA RÉALISATION DE TRAITEMENTS DIATHERMIQUES ET DE STIMULATION MUSCULAIRE

(30) Priority: 14.07.2021 IT 202100018503
(43) Date of publication of application: 22.05.2024
(73) Proprietor: BRERA MEDICAL TECHNOLOGIES SRL, 84061 Ogliastro Cilento (SA) (IT)
(72) Inventor: GRATTACASO, Attilio, 84043, Agropoli(SA) (IT)
(74) Representative: Conversano, Gabriele
(86) International application number: PCT/IB2022/055116
(87) International publication number: WO 2023/285887

(56) References cited:
- WO-A1-2013/103891
- IT-A1- NA20 110 019
- US-A- 4 249 537
- US-A1- 2017 189 227
- US-B2- 7 035 691

## Description

### Technical field

The present invention relates the technical field comprising devices for carrying out treatments on tissues and muscles, in particular diathermy and electrostimulation treatments.

Diathermy is a therapy consisting in increasing temperature of an inner body part by applying lowvoltage and high-voltage current, magnetic waves and ultrasound thereto; it is used for the treatment of some rheumatic muscle diseases. Instead, muscle electro-stimulators are made up of a low frequency electric current generator connected to a series of electrodes, and their usage is indicated to develop and strengthen muscles. The present invention relates to a device for treatments electro-stimulated by combined action of diathermy and muscle stimulation. More in particular, the present invention provides a device able to treat muscles by applying heat developed by diathermy combined with muscle electrostimulation.

### State of the art

Many kinds of devices for carrying out diathermy are known and commonly used at the state of the art, among which those described in documents US2017189227, WO2013103891, US4249437, ITNA20110019, which discloses the preamble of independent claim 1, and US7035691. Generally, these electromedical machines are operated by applying two electrodes, for example by using adhesive plates on the body area to be treated; in the following currents with intensity chosen according to the patient pathology are applied to the patient by means of said electrodes.

The currents applied develop, by Joule effect, an amount of heat in the patient's body, which is a function of the body impedance and so depends on the distance between the electrodes arranged on the body area to be treated.

The heat developed in this way will cause an increase in temperature, not superficial but localized in the deep layers, in the body area of the patient in the path between the two electrodes. The reached temperature is controlled by variation of the applied power and can be chosen according to the pathology to be treated.

Such treatment is very much used in sport rehabilitation, and it is often followed by an electrostimulation treatment in order to increase its effectiveness.

In fact, the body heating stimulates lipolysis, i.e. the metabolic process which provides triglycerides cleavage in free fatty acids and glycerol. These fatty acids are then consumed thanks to the muscle electrostimulation treatment, whose effectiveness is maximized by the previous diathermic treatment, since the duration of the electrostimulation treatment being equal, a heated muscle burns more fatty acids than a not heated muscle.

Today, diathermy is used in sport rehabilitation as preparatory step to electrostimulation in order to maximize its effects.

Combining diathermy with electrostimulation would be very convenient, in fact it would allow to treat each muscle faster than by the only electrostimulating effect. The treatment time reduction would allow obviously to treat more muscle groups in only one session, or, as an alternative to be able to treat more patients at the same time.

There exist devices combining the heat application with the electrostimulation in many ways.

For example, microwaves for heat generation are used together with the electrostimulation for muscle stimulation. Anyway, since microwaves are high frequency electromagnetic fields, there exists a risk linked to their usage both for operator and patient.

It is known in fact that the more the irradiation frequency increases, the more the interaction with the surrounding biological tissues is; moreover, microwaves have reduced penetration depth in tissues, since the most energy part is absorbed by the superficial layers.

There exist also devices able to carry out diathermy and electrostimulation, as for example US2021/0023364A1, which are however characterized by the need to use more discrete generators for each energy source.

Disadvantageously, these machines are provided with discrete couples of electrodes for each energy source and also use DC pulses for muscle stimulation (EMS), which, as known in literature, have clear limits of action range due to the high impedance of the superficial tissues at such frequencies.

Another limit of these devices is the fact that they are provided with a flat electrode surface, and so during the passage of the electrode on the concave and convex areas of the skin surface, a large part of the electrode surface loses contact with the skin.

In this case it occurs that, the irradiation power being equal, the energy density for surface unit of the electrode is greater, and so there occur overheating phenomena of the treated area with risk of burns. To avoid burns due to this problem, in the devices known at the state of the art, it is needed to use a lower electric intensity of the diathermic treatment than the one really needed. But, in this way, the heating application is mild and as a consequence the effectiveness of the treatment is remarkably reduced.

Another limit of the devices known at the state of the art is that the handpiece on which the electrode is installed which slides on the skin, since it is moved by an operator, has necessarily a not constant sliding speed on the body surface.

If such speed variation is very marked, since electrostimulation and diathermy parameters do not depend on the handpiece acceleration, it occurs that in the areas where speed is higher, the heat and electrostimulation application is lower, and vice versa.

Clearly, such aspect causes a lower treatment effectiveness in those areas where abrupt speed variations occur, and the treatment will depend substantially on the operator's skill in keeping the sliding speed constant without abrupt accelerations during the passage on the skin.

There exist also many models of muscle electro-stimulators which allow to maximize the treatment effectiveness.

An example is shown in US4249537A, which describes an apparatus to stimulate and control muscle contraction, which comprises conductive electrodes to contact positions at distance on the body. Another example is described in US7035691B2, which shows a method and device generating in the muscle resonant sequences having pulses at optimal intervals and configured to provide uniform contractions.

There exist also other known solutions which represent the usage of an electro-stimulator inside a temperature-controlled environment, in order to increase the effectiveness of the electrostimulation by means of heat application from outside.

According to a first aim, the present invention provides a mono-source device configured to electro-stimulate the muscle in a deep and combined way with a diathermic treatment.

According to another aim, the present invention provides a device configured to maximize the diathermic heat application while avoiding burns of the treated area, in order to obtain an effective vasodilation effect which increases muscle oxygenation and maximizes treatment effectiveness. According to another aim, the present invention provides a device configured to adapt the electric intensity parameters of the diathermic treatment and electrostimulation, while compensating the speed variations during the electrode sliding on the area to be treated.

According to another aim, the present invention provides a device configured to vary intensity and muscle heating depth so to be able to carry out a combined and optimized action of electrostimulation in independent way.

The invention is defined by the independent claim 1. The present invention realizes the prefixed aims since it is a device for carrying out diathermic and muscle stimulation treatments, comprising:
- a single power generator (1), to which there are connected a return electrode (2), and at the opposite pole, at least two electrodes (3), positioned on a handpiece (10) and separated from each other by means of insulating material (9),
- an electronic control unit (4) configured to manage the power supplied by said power generator (1) between said return electrode (2) and said at least two electrodes (3), characterized in that said electronic control unit (4) is configured to generate a whole voltage signal, made up of the combination of two functional signals, between the electrodes on the handpiece (3) and the return electrode (2);
- a first functional signal comprising a series of pulses (7) in the frequency spectrum generating diathermy;
- a second functional signal comprising a series of time variable amplitude radiofrequency pulses, for which the development of the maximum voltage of the pulse over time provides a waveform equal to the development of the action potential of muscle (8) able to overcome the skin resistance and to reach the deep layers,
said first and second signal being combined so that the amplitude thereof is controlled in independent way.

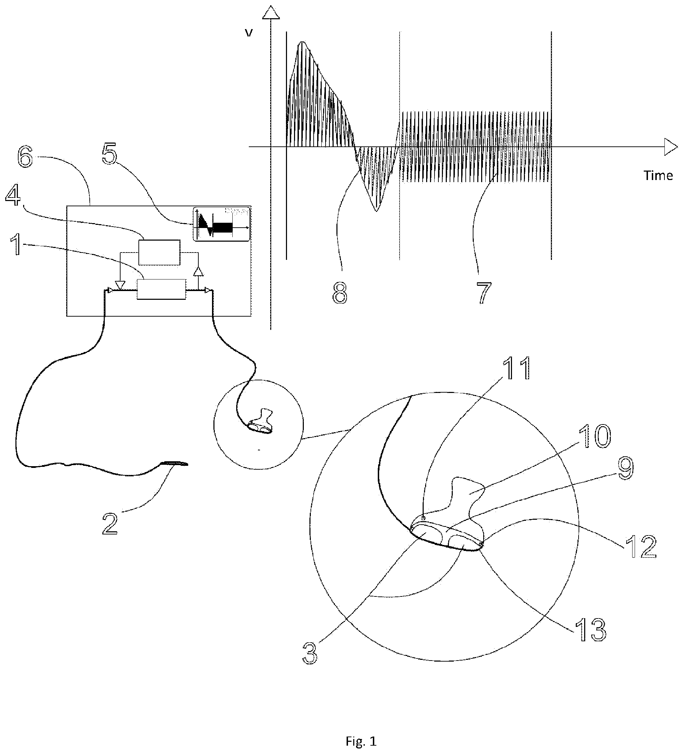

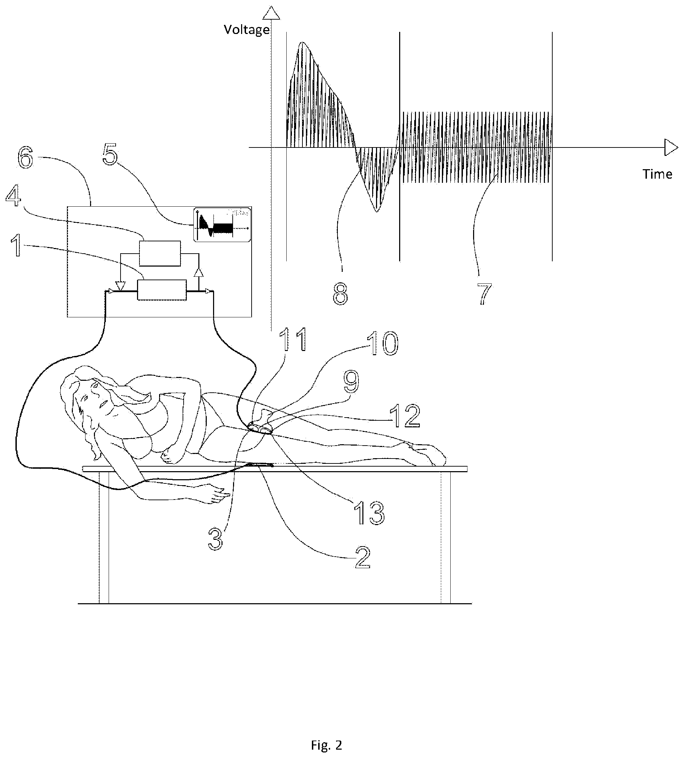

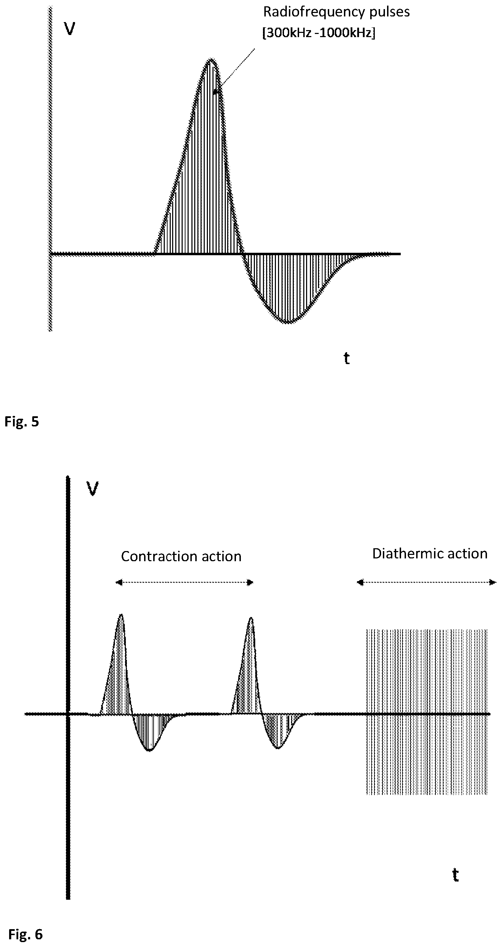

The device will be now described with reference to the appended figures 1 to 6. In figure 1, it is shown a schematic view of the main elements of the device; in figure 2, it is shown a treatment example by means of the device; in figure 3, on a time/applied voltage graph it is shown an example scheme of the signal used to carry out a diathermic treatment and whose values of applied voltage have constant peaks and null average value; in figure 4, it is shown the development of the time of muscle action potential; in figure 5, it is shown an action potential pulse obtained by the modulation of the radiofrequency portion, in figure 6, it is shown a combination example of the two above-described functional signals which produce a contraction effect and a diathermic effect in tissues.

With reference to what shown in the appended figures, in a preferred embodiment the device according to the invention comprises:
- a handpiece (10) provided with at least two electrodes (3) separated by insulating material (9) from each other;
- a single power generator (1) (shown inside a housing (6)), to which there are connected a return electrode (2) and, at the opposite pole, said at least two electrodes (3).

The device is configured so that the first electrode (2) can be positioned at contact with the skin at a muscle to be subjected to electrostimulation combined with diathermy, while said at least two electrodes (3) are positioned, by means of said handpiece (10), on the skin at the same muscle. The movement of the handpiece allows to carry out also a massage on such muscle.

The device comprises also an electronic control unit (4), electrically connected to the power generator (1) and configured to manage the power supplied by the power generator (1) between the first electrode (2) and said at least two electrodes (3).

The electronic control unit (4) is configured to generate a whole voltage signal, made up of the combination of two functional signals, between the electrodes on the handpiece (3) and the return electrode (2);
- a first functional signal comprising a series of pulses (7) in the frequency spectrum generating diathermy;
- a second functional signal comprising a series of time variable amplitude radiofrequency pulses, for which the development of the maximum voltage of the pulse over time provides a waveform equal to the development of the action potential of muscle (8), said first and second signal being combined so that the amplitude thereof is controlled in independent way.

It is to be specified that, with reference to the first signal, for frequency spectrum generating diathermy is intended the frequencies between 300 kHz and 1000 kHz. Moreover, preferably the average value of the signal is equal to zero, and the diathermic effect can be controlled by means of the signal amplitude (maximum voltage value).

It is to be specified instead, with reference to the second functional signal, that the repetition of this RF burst packet induces muscle contraction and the frequency thereof depends on the repetition over time of such packets.

The parameters which can be set are:
- the duration of each functional signal can vary between 20 us and 40 us;
- the repetition frequency of the second functional signal is between 0.5 Hz and 500 Hz;
- the pulses have frequency between 300 kHz and 1 MHz.

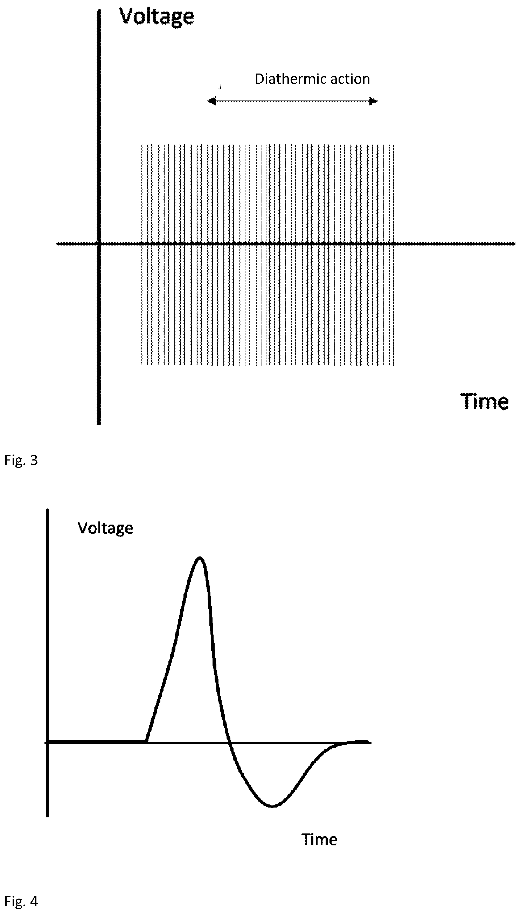

In the time intervals between one muscle contraction and the other one a diathermic signal can be sent, of the kind schematized in figure 3, and corresponding only to the first functional signal previously described.

Furthermore, the device comprises means for measuring the current flowing through each of said at least two electrodes (3) positioned on the handpiece, and said control unit (4) is configured to reduce the intensity of the diathermic treatment, by reducing the voltage of said first functional signal in a directly proportional way to the number of at least electrodes provided on the handpiece (3) for which the measured current intensity is null.

Thanks to this invention, it is possible to control the stimulation pulse independently of the diathermic one starting from a single power generator, in fact both the control of the electrostimulating pulse amplitude and of the diathermic action are extended. For example, by increasing the contraction pulse amplitude the contraction intensity is increased. By increasing the radiofrequency amplitude, the heat intensity transferred to the deep tissue is increased.

Moreover, preferably, to solve the technical problem of the abrupt speed variations linked to the not uniform sliding of the handpiece on the skin, and to make the treatment not operatordependent the handpiece (10) can be provided with at least an accelerometer (11) connected to the electronic control unit (4). The electronic control unit is configured to receive in input an acceleration signal detected by said accelerometer, to integrate such signal to obtain a speed signal and to increase the power supplied in a directly proportional way to the sliding speed of the handpiece on the treated area.

In this way, the heat application is maximized as a function of the sliding speed of the handpiece on the skin and the treatment does not depend on the operator's skill in keeping the sliding speed constant, but it is adapted to the treatment automatically.

At the insulating area (9) dividing the at least two electrodes (3) it is preferably provided at least a temperature sensor (12), configured to detect the skin temperature value and to intervene by controlling and adapting the power and by interrupting the treatment immediately in case the skin temperature exceeds a predetermined alarm threshold. Moreover, preferably, the surface of the handpiece (10), on which said at least two electrodes (3) are positioned, is provided with a curved edge in contact with the skin (13) so to be adapted to the concave and convex surfaces of the body. Thanks to the heat application due to the diathermic effect, there occurs a vasodilation, the muscle fibres slide with less friction and at the same time there is a greater oxygenation of the muscle with the consequent reduction of lactic acid formation. By using a traditional electro-stimulator, the muscle works anaerobically, this fact contributes to the formation of lactic acid which causes pain, and so it is needed to reduce the muscle working cycle with a typical ratio 1 to 5. As a consequence, it is normally used a lower stimulation with a reduced effect on the muscle. Moreover, by using a traditional electro-stimulator, which uses lower frequencies, the deeper muscle fasciae are not interested but only the surface is, on the contrary the generated contraction pulse, since it is a radiofrequency pulse, is able to interest the deep muscle fasciae since the tissue impedance is lower at such frequencies. Moreover, by varying the frequency of the functional signal (7) it is possible to act on the muscle heating depth, and so to carry out a combined and optimized action of electrostimulation, by acting instead on the amplitude and duration it is possible to manage the intensity of the action potential of the muscle (8) and so on the contraction intensity of the same muscle.

In a preferred embodiment, at least one of the electrode provided on the handpiece acts as return electrode.

In another preferred but not limiting embodiment, the device comprises a plurality of handpieces and a plurality of power generators (1), configured each to control a respective handpiece, so that a plurality of handpieces can function simultaneously.

## Claims

1. Device for carrying out diathermic and muscle stimulation treatments, comprising:
- a single power generator (1), to which there are connected a return electrode (2), and at the opposite pole, at least two electrodes (3), positioned on a handpiece (10) and separated from each other by means of insulating material (9),
- an electronic control unit (4) configured to manage the power supplied by said power generator (1) between said return electrode (2) and said at least two electrodes (3),
wherein
said electronic control unit (4) is configured to generate a whole voltage signal, made up of the combination of two functional signals, between the electrodes on the handpiece (3) and the return electrode (2):
- a first functional signal comprising a series of pulses (7) in the frequency spectrum generating diathermy;
- a second functional signal comprising a series of time variable amplitude radiofrequency pulses, for which the development of the maximum voltage of the pulse over time provides a waveform equal to the development of the action potential of muscle (8), said first and second signal being combined so that the amplitude thereof is controlled in independent way,
said device being **characterized in that**
it comprises also means for measuring the current flowing through each of said at least two electrodes (3) positioned on the handpiece and said control unit (4) is configured to adapt the intensity of the diathermic treatment, by varying the voltage of said first functional signal in a directly proportional way to the number of electrodes provided on the handpiece (3) for which the measured current intensity is null
and **in that**
it comprises at least one accelerometer (11) integral with said handpiece (10) and connected to said electronic control unit (4),
said electronic control unit being configured to receive in input the acceleration signal detected by said accelerometer, to integrate such signal to obtain a speed signal and to adjust the power supplied in a directly proportional way to the sliding speed of the handpiece on the treated area.

2. Device according to claim 1, **characterized in that** said first signal has a null average value and frequency between 300 kHz and 1000 MHz.

3. Device according to claim 1 or 2, **characterized in that** said second signal is **characterized by**:
- a duration of the waveform between 20 us and 40 us;
- a repetition frequency between 0.5 Hz and 500 Hz;
- and in that said radiofrequency pulses have frequency between 300 kHz and 1 MHz.

4. Device according to any one of the preceding claims, **characterized in that** said signal comprises a cyclic repetition of a signal deriving from the combination of said two functional signals.

5. Device according to any one of the preceding claims, **characterized in that** said handpiece (10) comprises also at least a temperature sensor (12) configured to detect the skin temperature value.

6. Device according to any one of the preceding claims, **characterized in that** the surface of the handpiece (10) on which said at least two electrodes (3) are positioned is provided with a curved edge (13) in contact with the skin.

7. Device according to any one of the preceding claims, **characterized in that** at least one of the electrodes provided on the handpiece acts as return electrode.

8. Device according to any one of the preceding claims, comprising a plurality of handpieces and a plurality of power generators (1), configured each to control a respective handpiece, so that a plurality of handpieces can function simultaneously.

## Patentansprüche

1. Vorrichtung zur Durchführung von Diathermie- und Muskelstimulationsbehandlungen, umfassend:
- einem einzigen Stromgenerator (1), an den eine Rücklaufelektrode (2) und am Gegenpol mindestens zwei Elektroden (3) angeschlossen sind, die auf einem Handstück (10) angeordnet und durch ein Isoliermaterial (9) voneinander getrennt sind,
- eine elektronische Steuereinheit (4), die dazu konfiguriert ist, die von dem Stromgenerator (1) zwischen der Rücklaufelektrode (2) und den mindestens zwei Elektroden (3) gelieferte Leistung zu verwalten, wobei die elektronische Steuereinheit (4) dazu konfiguriert ist, zwischen den Elektroden am Handstück (3) und der Rücklaufelektrode (2) ein Gesamtspannungssignal zu erzeugen, das aus der Kombination zweier Funktionssignale besteht;
- ein erstes Funktionssignal, das aus einer Reihe von Impulsen (7) im Diathermie-erzeugenden Frequenzspektrum besteht;
- ein zweites Funktionssignal, das eine Reihe von Radiofrequenzimpulsen mit zeitlich veränderlicher Amplitude umfasst, bei denen die zeitliche Entwicklung der maximalen Spannung des Impulses eine Wellenform ergibt, die der Entwicklung des Aktionspotentials des Muskels (8) entspricht, wobei das erste und das zweite Signal so kombiniert sind, dass ihre Amplitude unabhängig voneinander gesteuert werden kann, wobei die Vorrichtung **dadurch gekennzeichnet ist, dass** sie außerdem Mittel zum Messen des Stroms umfasst, der durch jede der mindestens zwei Elektroden (3) fließt, die auf dem Handstück angeordnet sind, und dass die Steuereinheit (4) so konfiguriert ist, dass sie die Intensität der diathermischen Behandlung anpasst, indem sie die Spannung des ersten Funktionssignals in einer Weise ändert, die direkt proportional zur Anzahl der Elektroden ist, die auf dem Handstück (3) angeordnet sind, für die die gemessene Stromstärke Null ist, und dass sie mindestens einen Beschleunigungsmesser (11) umfasst, der in das Handstück (10) integriert ist und mit der elektronischen Steuereinheit (4) verbunden ist, wobei die elektronische Steuereinheit so konfiguriert ist, dass sie als Eingangssignal das von dem Beschleunigungsmesser erfasste Beschleunigungssignal empfängt, um das Signal zu integrieren, ein Geschwindigkeitssignal zu erhalten, und die zugeführte Leistung in einer Weise anzupassen, die direkt proportional zur Gleitgeschwindigkeit des Handstücks auf der behandelten Oberfläche ist.

2. Vorrichtung nach Anspruch 1, **dadurch gekennzeichnet, dass** das erste Signal einen Mittelwert von Null und eine Frequenz zwischen 300 kHz und 1000 MHz aufweist.

3. Vorrichtung nach Anspruch 1 oder 2, **dadurch gekennzeichnet, dass** das zweite Signal **gekennzeichnet ist durch**:
- eine Wellenformdauer zwischen 20 us und 40 us;
- eine Wiederholfrequenz zwischen 0,5 Hz und 500 Hz;
- und dass die Hochfrequenzimpulse eine Frequenz zwischen 300 kHz und 1 MHz aufweisen.

4. Vorrichtung nach einem der vorhergehenden Ansprüche, **dadurch gekennzeichnet, dass** das Signal eine zyklische Wiederholung eines Signals umfasst, das aus der Kombination der beiden Funktionssignale abgeleitet ist.

5. Vorrichtung nach einem der vorhergehenden Ansprüche, **dadurch gekennzeichnet, dass** das Handstück (10) außerdem mindestens einen Temperatursensor (12) umfasst, der zum Erfassen des Werts der Hauttemperatur konfiguriert ist.

6. Vorrichtung nach einem der vorhergehenden Ansprüche, **dadurch gekennzeichnet, dass** die Oberfläche des Handstücks (10), auf der die mindestens zwei Elektroden (3) angeordnet sind, mit einer gekrümmten Kante (13) versehen ist, die mit der Haut in Kontakt steht.

7. Vorrichtung nach einem der vorhergehenden Ansprüche, **dadurch gekennzeichnet, dass** mindestens eine der am Handstück vorgesehenen Elektroden als Rücklaufelektrode fungiert.

8. Vorrichtung nach einem der vorhergehenden Ansprüche, das mehrere Handstücke und mehrere Energiegeneratoren (1) umfasst, die jeweils so konfiguriert sind, dass sie ein entsprechendes Handstück steuern, so dass mehrere Handstücke gleichzeitig betrieben werden können.

## Revendications

1. Dispositif pour réaliser des traitements de diathermie et de stimulation musculaire, comprenant:
- un générateur de puissance unique (1), auquel sont connectées une électrode de retour (2), et au pôle opposé, au moins deux électrodes (3), positionnées sur une pièce à main (10) et séparées l'une de l'autre par un matériau isolant (9),
- une unité de commande électronique (4) configurée pour gérer la puissance fournie par ledit générateur de puissance (1) entre ladite électrode de retour (2) et lesdites au moins deux électrodes (3), dans lequel ladite unité de commande électronique (4) est configurée pour générer un signal de tension totale, constitué de la combinaison de deux signaux fonctionnels, entre les électrodes sur la pièce à main (3) et l'électrode de retour (2);
- un premier signal fonctionnel comprenant une série d'impulsions (7) dans le spectre de fréquence générant la diathermie;
- un deuxième signal fonctionnel comprenant une série d'impulsions radiofréquences d'amplitude variable dans le temps, pour lesquelles l'évolution de la tension maximale de l'impulsion au cours du temps fournit une forme d'onde égale à l'évolution du potentiel d'action du muscle (8), lesdits premier et deuxième signaux étant combinés de manière à ce que leur amplitude soit contrôlée de manière indépendante, ledit dispositif étant **caractérisé en ce qu'**il comprend aussi des moyens pour mesurer le courant qui passe à travers chacun des dites au moins deux électrodes (3), positionnées sur la pièce à main, et la dite unité de commande (4) étant configurée pour adapter l'intensité du traitement diathermique, en modifiant la tension dudit premier signal fonctionnel dans une manière directement proportionnelle au nombre des électrodes fournies sur la pièce à main (3) pour lesquelles l'intensité de courant mesurée est nulle, et **en ce qu'**il comprend au moins un accéléromètre (11) intégré dans ladite pièce à main (10) et il est connecté à ladite unité de commande électronique (4), ladite unité de commande électronique étant configurée pour recevoir en entrée le signal d'accélération détecté par ledit accéléromètre, pour intégrer ledit signal en obtenant un signal de vitesse et pour régler la puissance fournie dans une manière directement proportionnelle à la vitesse de glissement de la pièce à main sur la surface traitée.

2. Dispositif selon la revendication 1, **caractérisé en ce que** ledit premier signal présente une valeur moyenne nulle et une fréquence comprise entre 300 kHz et 1000 MHz.

3. Dispositif selon la revendication 1 ou 2, **caractérisé en ce que** ledit deuxième signal est **caractérisé par**:
- une durée de la forme d'onde comprise entre 20 us et 40 us ;
- une fréquence de répétition comprise entre 0,5 Hz et 500 Hz;
- et en ce que lesdites impulsions radiofréquences présentent une fréquence comprise entre 300 kHz et 1 MHz.

4. Dispositif selon l'une quelconque des revendications précédentes, **caractérisé en ce que** ledit signal comprend une répétition cyclique d'un signal dérivant de la combinaison desdits deux signaux fonctionnels.

5. Dispositif selon l'une quelconque des revendications précédentes, **caractérisé en ce que** ladite pièce à main (10) comprend également au moins un capteur de température (12) configuré pour détecter la valeur de la température de la peau.

6. Dispositif selon l'une quelconque des revendications précédentes, **caractérisé en ce que** la surface de la pièce à main (10) sur laquelle sont positionnées lesdites au moins deux électrodes (3) est pourvue d'un bord incurvé (13) en contact avec la peau.

7. Dispositif selon l'une quelconque des revendications précédentes, **caractérisé en ce qu'**au moins une des électrodes prévues sur la pièce à main agit comme une électrode de retour.

8. Dispositif selon l'une quelconque des revendications précédentes, comprenant une pluralité de pièces à main et une pluralité de générateurs d'énergie (1), configurés chacun pour commander une pièce à main respective, de sorte qu'une pluralité de pièces à main peuvent fonctionner simultanément.
